(19) Europäisches Patentamt
European Patent Office
Office européen des brevets

(11) **EP 3 831 307 A1**

(12) **EUROPEAN PATENT APPLICATION**

(43) Date of publication:
**09.06.2021 Bulletin 2021/23**

(21) Application number: **19212768.6**

(22) Date of filing: **02.12.2019**

(51) Int Cl.:
*A61B 8/12* (2006.01)      *A61B 8/08* (2006.01)
*A61B 8/06* (2006.01)      *A61B 5/02* (2006.01)
*A61B 5/021* (2006.01)      *A61B 5/0215* (2006.01)
*A61B 8/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **VAN DER HORST, Arjen**
**5656 AE Eindhoven (NL)**
• **KUENEN, Maarten Petrus Joseph**
**5656 AE Eindhoven (NL)**

(74) Representative: **Philips Intellectual Property & Standards**
**High Tech Campus 5**
**5656 AE Eindhoven (NL)**

(54) **AN APPARATUS FOR DETERMINING A PHYSIOLOGICAL PARAMETER RELATING TO A VESSEL**

(57)      An apparatus for determining a physiological parameter relating to a vessel of a subject comprises flow and pressure sensors located at a distance from each other. A forward looking ultrasound transducer provides flow measurement and the pressure sensor is distal from the ultrasound transducer. A physiological parameter is derived based on blood flow velocity measurement and pressure measurement at the same location of the vessel.

FIG. 2

**Description**

FIELD OF THE INVENTION

The invention relates to the field of vascular assessment, and more specifically to the field of characterizing vessels.

BACKGROUND OF THE INVENTION

Arterial stiffening is an important risk factor for cardiovascular disease. Arterial stiffness increases with aging and various disease states, including hypertension, hypercholesterolemia, diabetes mellitus, obesity, smoking, and kidney disease.
A commonly used parameter to assess the stiffness of vessel is pulse wave velocity (PWV). PWV is the transmission speed of pressure/flow waves (e.g. generated by the beating heart) through the arteries. The PWV is determined by the ability of the vessel to expand (i.e. distensibility, D) according to the following relation:

$$PWV = \sqrt{\frac{1}{\rho D}} \text{, with} \qquad D = \frac{dV}{VdP} \qquad (1)$$

With V the vascular volume, P the pressure, and $\rho$ the blood density. From this relation, the Moens-Korteweg equation can be derived:

$$PWV = \sqrt{\frac{E \cdot h}{d \cdot \rho}} \qquad (2)$$

Here, E is the Young's modulus, d is the vessel diameter, and h is the wall thickness. By assessing the PWV, the stiffness of arteries can therefore be quantified.
As the waves travel very quickly through the vessels, most commonly the PWV is determined over relatively large distances in the vasculature, e.g. brachial artery to ankle. In this way, the average stiffness of the vessels in between the measurement locations is determined.
In the last decade, more local PWV measurements have been developed. For superficial arteries external ultrasound can for example be used to assess PWV. Another approach, that is also suitable for local assessment of the PWV in deeper arteries, is based on the use of sensor-equipped catheters, guidewires or other minimally invasive devices.
For example, two or multiple pressure sensors on a catheter at known distances (x) can be used to determine the time difference ($\Delta t$) of the passing waves: $\left(PWV = \dfrac{x}{\Delta t}\right)$. However, at a nominal PWV of approximately 10 m/s, the time difference to be measured becomes very short when measuring in short arteries. This poses significant challenges to the measurement requirements, in particular a higher sampling frequency than would normally be required for standard non-PWV measurements is needed.
Another way to determine the PWV is by utilizing the "water hammer" equation (also known as the slope method) to calculate the PWV from simultaneous pressure (P) and flow velocity (U) measurements inside:

$$PWV = \frac{1}{\rho}\frac{dP}{dU} \qquad (3)$$

Alternatively, the following relation can be used that determines the PWV by summation over the whole cardiac cycle:

$$PWV = \frac{1}{\rho}\sqrt{\frac{\sum dP^2}{\sum dU^2}} \qquad (4)$$

This slope method is described in further detail in Davies et al, 2006, "Use of simultaneous pressure and velocity measurements to estimate arterial wave speed at a single site in humans", Am J Physiol Heart Circ Physiol. 290(2):H878-85.

The PWV can be determined via Equations 3 or 4 above. The advantage of this is that the sampling frequency does not need to be so high. A disadvantage is that the pressure and flow velocity need to be measured at the same location.

There remains a need for a sampling apparatus which in a simple manner obtain the flow velocity and pressure measurements from a single location.

SUMMARY OF THE INVENTION

The invention is defined by the claims.

According to examples in accordance with an aspect of the invention, there is provided an apparatus for determining a physiological parameter relating to a vessel of a subject, the apparatus comprising:

a catheter for insertion into the vessel;
an ultrasound transducer having a first position which is, in use, set relative to a distal end of the catheter, wherein the ultrasound transducer is forward looking;
a pressure sensor having a second position which is, in use, set relative to the distal end of the catheter and is distal from the ultrasound transducer; and
a processor,
wherein the processor is adapted to:
determine from the pressure sensor output and the ultrasound transducer output a time point at which, or a time period during which, the parameter measurement should be obtained; and
at the determined time point or over the time period:

determine an ultrasound Doppler blood flow velocity measurement from the ultrasound transducer output relating to the second position; and
process the pressure sensor output and the blood flow velocity measurement to derive the physiological parameter measurement relating to the vessel.

This apparatus mounts a forward looking ultrasound probe at the end of a catheter, with a pressure sensor further forward (i.e. more distal). The two sensors are in use positioned in set positions, such that the location of the flow velocity measurement and the location of the pressure measurement align. This enables accurate parameter estimation of physiological parameters like PWV (e.g. using the so-called slope method), impedance or resistance, and vasomotion.

Note that the apparatus may be inserted in the flow direction (so that the pressure sensor is downstream of the ultrasound transducer) or it may be inserted in the opposite direction with the flow facing the distal end of the catheter. The pressure sensor is then upstream from the ultrasound transducer in terms of the blood flow. The term distal refers to the end of the catheter in the body, and the term proximal refers to the end of the catheter outside the body.

The processor is preferably adapted to determine from the pressure sensor output and/or the ultrasound transducer output a time point comprising a specific part of a cardiac cycle.

This time point is for example a reflection-free period, such as the early systole.

The processor may be adapted to determine from the pressure sensor output and the ultrasound transducer output a time period of a complete cardiac cycle.

If a reflection free period cannot be used, a summation over the complete cardiac cycle may be used.

The pressure and flow may for example be analyzed in certain frequency ranges - e.g. 0.05-1 Hz related to vasomotion.

The processor may be adapted to derive a blood flow velocity measurement at the location of the pressure sensor. This enables a most accurate parameter determination from simultaneous pressure and flow velocity measurements.

The processor may be adapted to tune electronically a sampling distance of the blood flow velocity from the ultrasound transducer to correspond to the location of the pressure sensor. The ultrasound transducer may in this way be tuned to the known distance between the ultrasound transducer and the more distal pressure sensor. This enables accurate positional alignment of the pressure and flow velocity signals.

In a first set of examples, the apparatus comprises a guidewire along which the catheter is guided, wherein the ultrasound transducer is mounted at a distal end of the catheter and the pressure sensor is mounted at distal end of the guidewire, which projects beyond the distal end of the catheter.

This first arrangement makes use of a guidewire and catheter combination. The guidewire extends beyond the distal end of the catheter in use.

The ultrasound transducer is for example radiopaque or echogenic and the guidewire comprises a radiopaque or echogenic marker for alignment relative to the ultrasound transducer. In this way, the first and second positions can be set, using angiography or ultrasound.

The catheter may instead comprise a radiopaque or echogenic marker at the distal end and the guidewire comprises a radiopaque or echogenic marker for alignment relative to the radiopaque or echogenic marker of the catheter.

Radiopaque markers may be used for x-ray angiography, and echogenic markers may be used for ultrasound guidance. In an example the marker on the catheter and the marker on the guidewire have to partially or completely overlap for a predetermined alignment. Depending on the configuration, the markers of the two intravascular devices (e.g. two guidewires, one having the ultrasound transducer and one the pressure sensor) or at least a portion of the markers have to be located in a same transversal plane of the vessel.

In another implementation, the guidewire comprises a marker at the proximal end for alignment relative to the catheter using visual inspection at the proximal end. In this way, the sliding of the catheter over the guidewire at the proximal end is conducted until the first and second positions are set, without the need for image guidance.

The catheter may comprise a locking arrangement for locking the relative positions of the catheter and the guidewire. In this way, the first and second positions can be fixed. This for example enables the assembly to be moved along a vessel as a combined unit, for parameter determination along the vessel, for creating a parameter map.

In a second set of examples, the apparatus may comprise:

a first guidewire along which the catheter is guided, wherein the pressure sensor is mounted at distal end of the first guidewire;
a second guidewire along which the catheter is guided, wherein the ultrasound transducer is mounted at a distal the end of the second guidewire.

This provides a two-guidewire assembly. The catheter ensures the correct relative positioning of the two guidewires.

The first and second guidewires may each comprise a radiopaque or echogenic marker at the distal end.

The catheter may additionally or alternatively comprise a locking arrangement for locking the relative positions of the first and second guidewires.

In a third set of examples, the pressure sensor is mounted on an extension which projects beyond the distal end of the catheter. This enables insertion of a modified catheter, which includes both sensors. Thus, it does not require relative alignment to be adjusted between a guidewire and a catheter.

In all examples, the physiological parameter may be the pulse wave velocity, resistance, (complex) impedance, or vasomotion.

The apparatus may further comprise a display device, wherein the processor is adapted to generate an image of the vessel and a parameter representation for display by the display device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a known apparatus for measurement of flow velocity and pressure inside a vessel;
Figure 2 shows the sensor part of an example of an apparatus in accordance with the invention;
Figure 3 shows the overall apparatus or system, comprising the sensor part of Figure 2 and processing circuitry;
Figure 4 shows an example of an ultrasound transducer tipped catheter;
Figure 5 shows how electronic fine tuning of the ultrasound transducer enables the sampling location to be aligned with the pressure sensor location;
Figure 6 shows another example having first and second guidewires;
Figure 7 shows an example in which the pressure sensor is mounted on an extension which projects beyond the distal end of the catheter; and
Figure 8 shows an example of the flow catheter, for example as used in Figure 2 with exemplary dimensions.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for determining a physiological parameter relating to a vessel of a subject which has a catheter for insertion into the vessel. A forward looking ultrasound transducer is at first position relative to a distal end of the catheter and a pressure sensor is distal from the ultrasound transducer. A physiological parameter is derived based on ultrasound Doppler blood flow velocity measurement and pressure measurement at the same location.

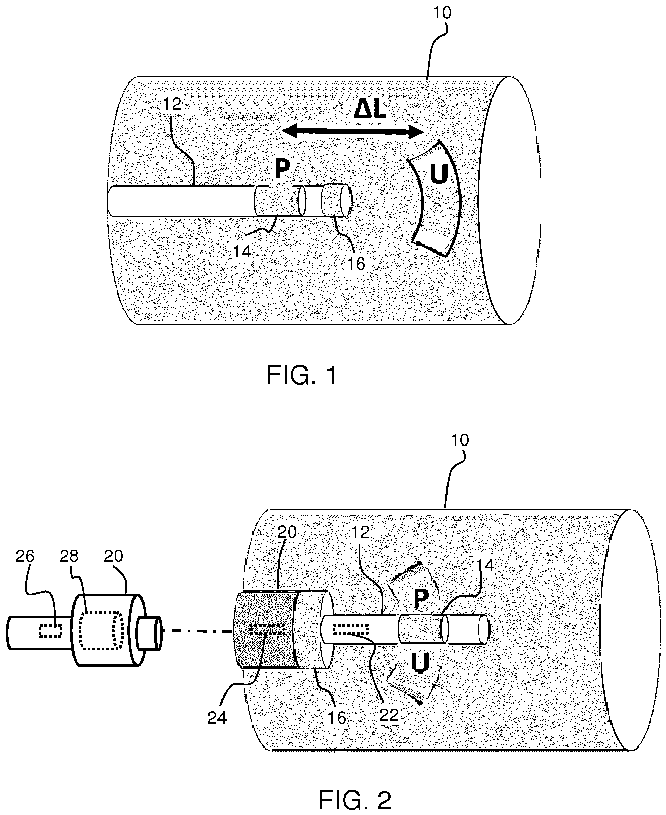

Figure 1 shows schematically a known apparatus for measurement of flow velocity and pressure inside a vessel 10, such as the ComboWire from Philips Volcano. The apparatus comprises a guidewire 12 with a pressure sensor 14 and an ultrasound Doppler flow velocity sensor 16 at the tip. The ultrasound Doppler flow velocity sensor 16 is located at the tip and it is forward looking, hence located more distal than the pressure sensor 14.

The pressure (P) and flow velocity (U) measurements are not sampled at the same location but there is a distance ($\Delta L$) up to 1 cm between the location where the pressure and flow velocity are measured. In very short branching arteries, e.g. renal arteries, this distance $\Delta L$ will make a derived PWV, impedance/resistance or vasomotion measurement less accurate and also it is more difficult to find an appropriate measurement location.

Figure 2 shows the sensor part of an example of an apparatus in accordance with the invention. The apparatus is generally for determining a physiological parameter relating to a vessel of a subject.

One preferred implementation is for determining the pulse wave velocity. Other examples are the resistance or impedance (including the frequency dependence) and based on that vasomotion.

The apparatus comprises a catheter 20 for insertion into the vessel 10.

An ultrasound transducer 16 has a first position which is, in use, set relative to a distal end of the catheter 20. The ultrasound transducer is forward looking.

A pressure sensor 14 has a second position which is, in use, also set relative to the distal end of the catheter 20, but it is distal from the ultrasound transducer 16.

It is for example between 3 mm and 1.5 cm more distal than the ultrasound transducer.

The advantage of measuring not too close to the ultrasound transducer is to allow the ringdown of the transducer to dampen and the disturbance of the flow caused by the distal end of catheter to minimize. A distance limit of 1.5 cm is for example still allowable as a result of the attenuation of the high frequency ultrasound, which means that beyond 1.5 cm a significantly reduced signal will be received back at the ultrasound transducer.

The flow velocity measurement obtained by the ultrasound transducer is in front of the transducer itself and it is designed to overlap in space with the location of the pressure sensor (e.g. the flow velocity measurement in the same transversal plane of the vessels as the pressure measurement).

This apparatus in this way mounts a forward looking ultrasound probe at the distal portion of a catheter (e.g. at the distal end of the catheter), with a pressure sensor more distal. The two sensors are in use positioned in set positions, such that the location of the flow velocity measurement and the location of the pressure measurement align in a transversal plane (i.e. radial to the longitudinal axis of the vessel and/or the catheter 20 - guidewire 12 assembly). This enables accurate PWV parameter estimation using the so-called slope method.

The guidewire stabilizes the orientation of the catheter with respect to the vessel axis, which makes the measurement less orientation dependent, especially during a pullback measurement (used to map the parameter over the length of the vessel).

In the example of Figure 2, the apparatus comprises a guidewire 12 along which the catheter 20 is guided. Catheters and guidewires are intravascular devices. The ultrasound transducer 16 is mounted at the distal end of the catheter 20 whereas the pressure sensor is mounted at distal portion of the guidewire 12, which projects beyond the distal end of the catheter.

In a first arrangement, the ultrasound transducer 16 is radiopaque (or echogenic) and the guidewire comprises a radiopaque (or echogenic) marker 22 for alignment relative to the ultrasound transducer. In this way, the first and second positions can be set, using angiography (or ultrasound).

The catheter may instead comprise a radiopaque (or echogenic) marker 24 at the distal end for alignment relative to the radiopaque (or echogenic) marker 22 of the catheter.

In another implementation, the guidewire 12 comprises a marker 26 at the proximal end for alignment relative to the catheter using visual inspection at the proximal end. In this way, the sliding of the catheter over the guidewire at the proximal end is conducted until the first and second positions of the sensors are set (e.g. the markers at least partially overlap), without the need for image guidance. This is appropriate for an "over the wire" catheter, where the guidewire is inside the catheter lumen over the entire length.

Thus, there may be only the marker 22, or the pair of markers 22 and 24, or only the marker 26. There may instead be a combination of different markers to enable different inspection approaches.

The catheter may also comprise a locking arrangement 28 for locking the relative positions of the catheter 20 and the guidewire 12. In this way, the first and second positions of the sensors can be fixed. This for example enables the assembly to be moved along a vessel as a combined unit, for parameter determination along the vessel, for creating a parameter map. The locking mechanism may be electronically or mechanically operated. The electronically operated locking mechanism may comprise a portion of electroactive polymer inner lumen wall of the catheter that upon application of electrical energy grips the outer surface of the guidewire, or increases the friction to such extent that the catheter and guidewire form a locked combined unit. Alternatively, mechanical locking mechanisms known in the art can be used.

To improve the accuracy of alignment of markers, especially when the tips of the devices are not perpendicular to the angiography imaging plane, multiple radiopaque or echogenic markers can be placed (on one or both devices) to make the alignment more accurate.

Figure 2 thus shows various options for ensuring correct alignment between the pressure sensor and the flow velocity sensor. They may be used in various combinations, so in some applications they will not be present.

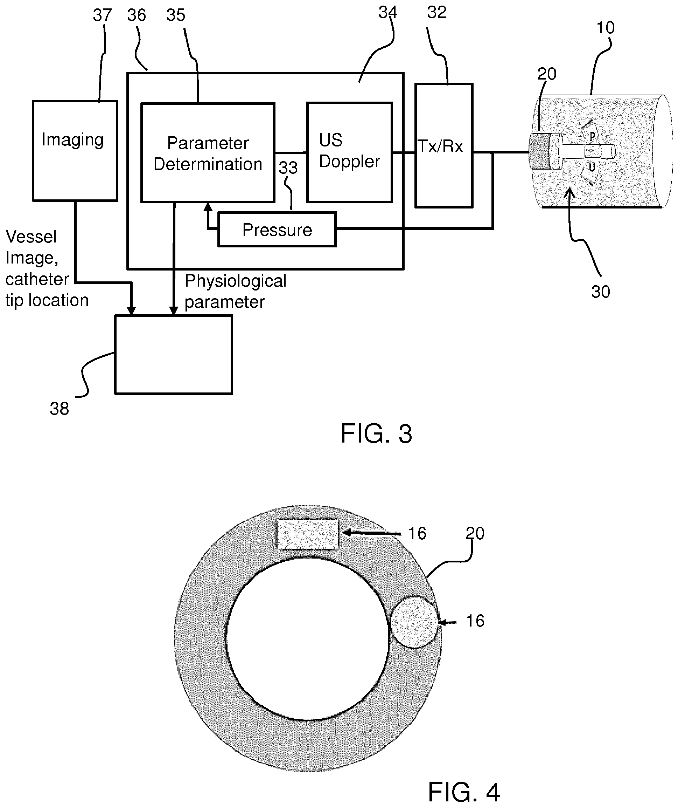

Figure 3 shows the overall apparatus or the system, comprising the sensor part 30 as described above, an ultrasound transmission and reception circuit 32, and a processor 36 which includes an ultrasound processing circuit 34, a pressure processing unit 33 and a determination unit 35 which determines the physiological parameter. The ultrasound transmission and reception circuit 32 transmits and receives the ultrasound signals. The processing circuit 34 processes the ultrasound data into a flow velocity spectrum for display and optionally an image or velocity map.

An imaging system 37 (such as an angiography or an ultrasound imaging device) enables creation of a representation of the parameter with a known location relative to the anatomical structure so that they may be represented together. It also represents the imaging of the vessel (although this may be based on imaging at the catheter tip, when an imaging catheter is used).

A display device 38 is used to display an image of the vessel and a parameter representation.

The processor 36 is adapted to determine from the pressure sensor output and the ultrasound transducer output a time point at which, or a time period during which, the parameter measurement should be obtained.

This is performed in order to obtain the flow velocity and pressure measurements at a desired point in time within the cardiac cycle, or to obtain a measurement over the duration of one or more complete cardiac cycles. The desired point in time is for example a reflection-free period, associated to early systole (e.g. first quarter or first half of the systole). In some cases where a reflection free period is not used, a summation over the complete cardiac cycle or several cycles may be used. The cardiac cycle information is obtained from the fluctuation in the pressure signal and/or the flow velocity signal. In principle, the duration of the cardiac cycle can be determined from the pressure measurements that vary cyclically with the heartbeat, or from the blood flow velocity measurement obtained by the ultrasound transducer.

At the determined time point or over the time period an ultrasound Doppler blood flow velocity measurement is derived from the ultrasound transducer output. This flow velocity measurement relates to the second position, namely the position at which the pressure sensor is located. The distance from the ultrasound sensor on the catheter to the transversal plane in which the pressure sensor is located, can also be called sampling distance of the ultrasound transducer.

The processor 36 thereby synchronizes the collection of both the pressure and ultrasound data, and receives the synchronized pressure and ultrasound data.

The processing of the ultrasound signals enables the location at the which the flow velocity measurement is taken to be selected. For example, the processor preferably tunes electronically the sampling distance of the blood flow velocity from the ultrasound transducer to correspond to the location of the pressure sensor. The ultrasound transducer is in this way fine tuned to the known distance between the ultrasound transducer and the more distal pressure sensor, and the blood flow velocity measurement is associated to the sampling distance, at the location of the pressure sensor. Alternatively, or additionally, the sampling distance is determined from the known configuration of the catheter and the guidewire, in particular the location of the two sensors on the respective devices. In a further alternative or additional embodiment, the sampling distance is related to the configuration of the catheter and guidewire in combination with the positions of the markers comprised on the respective devices and which are visualized on an imaging modality by an extracorporeal imaging device (e.g. x-ray based imaging, ultrasound imaging).

The synchronized and spatially aligned pressure sensor output and the blood velocity measurement are processed to derive the physiological parameter measurement relating to the vessel, such as the PWV, complex impedance, resistance, vasomotion. This is for example according to Equations 3 or 4 above.

In an embodiment the ultrasound transducer on the distal portion of the catheter is configured to provide ultrasound signal representative of its distance to the pressure sensor. This can be achieved by ultrasound imaging, wherein the pressure sensor or its location comprises a marker that is visible on the ultrasound image (e.g. echogenic marker). The ultrasound imaging may be performed sequentially or simultaneously with the pressure and flow velocity measurement. In an example the ultrasound transducer is an annular forward looking transducer on the catheter, wherein some ultrasound transducer elements are used for ultrasound imaging while others are used for flow velocity measurement. In other example, the ultrasound imaging is preformed prior the flow velocity and pressure measurements with the same ultrasound transducer as used for blood flow velocity measurement (e.g. by switching operation mode of the ultrasound transducer), and the sampling distance is determined by the processor automatically from the ultrasound image, which sampling distance is then used for computing the physiological parameter such as PWV, complex hydraulic impedance, hydraulic resistance, vasomotion.

Figure 4 shows an example of an ultrasound transducer tipped catheter 20 for advancing over the guidewire 12. The transducer can be in an annular form with at least one forward looking (axially distal facing) transducer element as shown in Figure 2, or a single or multiple elements at the tip of the catheter as shown in Figure 4. The transducer can for example comprise piezoelectric elements, micromachined ultrasound elements (CMUT or PMUT) or combination thereof.

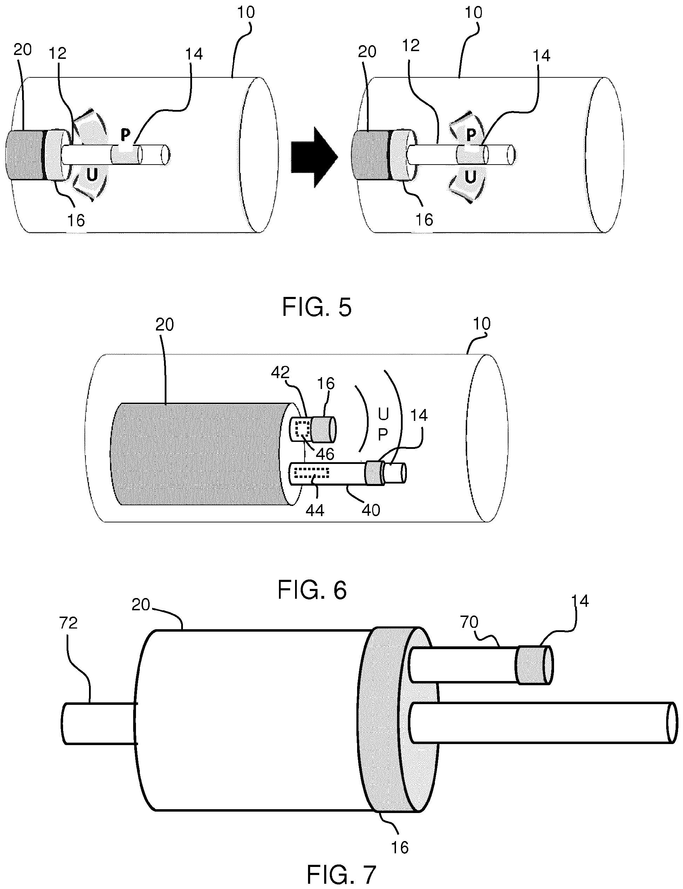

Figure 5 shows how the electronic fine tuning of the ultrasound transducer enables the sampling location to be aligned with the pressure sensor location.

Figure 6 shows another example having a first guidewire 40 along which the catheter is guided, wherein the pressure sensor 14 is mounted at distal end of the first guidewire. A second guidewire 42 along which the catheter is also guided has the ultrasound transducer 16 mounted at its distal end.

This provides a two-guidewire assembly. The catheter 20 ensures the correct relative positioning of the two guidewires.

The first and second guidewires may each comprise a radiopaque or echogenic marker 44, 46 at their distal end. This arrangement may also have a locking arrangement for locking the relative positions of the first and second guidewires.

Figure 7 shows an example in which the pressure sensor 14 is mounted on an extension 70 which projects beyond the distal end of the catheter 20. The pressure sensor can be proximal from the tip of the extension rather than at the very end as shown, to enable a soft tip of the extension. The ultrasound transducer 16 is at the end of the catheter. This enables insertion of a modified catheter, which includes both sensors. Thus it does not require relative alignment to be adjusted between a guidewire and a catheter. The catheter is for example inserted using a guidewire 72.

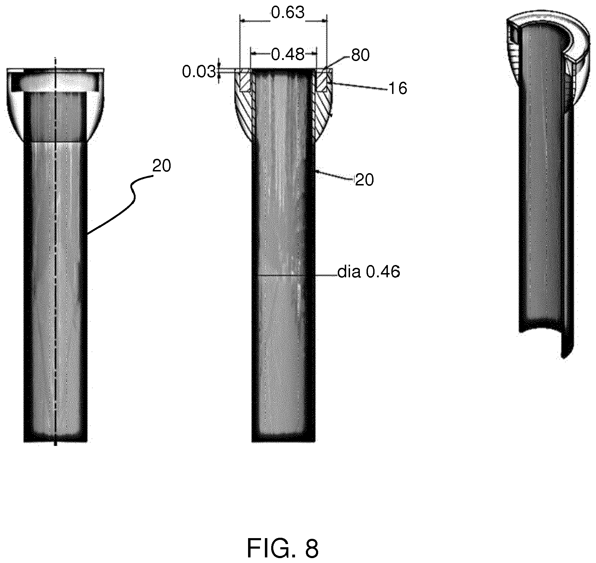

Figure 8 shows an example of the flow measurement catheter, for example as used in Figure 2, for guiding over a guidewire which as the distal pressure sensor. Example dimensions are shown, in mm. The ultrasound transducer is shown as an annular ring at the distal end of the catheter 20 (with an acoustic window material of 0.03 mm thickness).

The apparatus may for example be used to within the renal arteries to measure the renal blood pressure. For example, the PWV inside the main renal artery may be used as a predictor of the outcome of renal denervation treatment in patients with resistive hypertension. Quantitatively, pulse wave velocity measurements larger than approximately 10 m/s may be taken as an indication that a patient is likely to respond to renal denervation therapy focusing on interruption of the efferent and afferent signals that stimulate the renin-angiotensin-aldosterone system and regulate whole-body sympathetic nervous system activity. The renal denervation therapy can be achieved for example by ablation techniques of nerves located beyond the inner wall of the renal artery through applying energy with intravascular devices (e.g. radio frequency energy, ultrasound energy, etc). The apparatus may be adapted to determine from the pressure and flow measurements at the same location whether the pulse wave velocity of the renal artery is larger than a predetermined threshold, for example of 10 m/s, in order to automatically determine eligibility of subjects for renal denervation therapy. In other example, low coronary PWV has been associated with acute coronary syndromes, indicating a relation between plaque vulnerability and arterial stiffness. The apparatus may similarly be adapted to automatically stratify the subjects according to plaque vulnerability.

In some examples the ultrasound transducer may comprise CMUT transducers; piezoelectric transducers, formed of materials such as PZT or PVDF; or any other suitable transducer technology. The pressure sensors may be piezoresistive, capacitive or optical such as based on a Fabry-Perot interferometer.

An ultrasound pulse is transmitted from the transducer and after time $\Delta t$ the transducer receives the backscattered ultrasound waves. By analysis of the received ultrasound signals, the blood velocities can be deduced as in standard ultrasound pulsed Doppler methods. From the time between transmission and reception of the backscattered ultrasound waves, the distance between the transducer tip and the scattering objects can be selected.

A single transducer is used in the examples above. If a transducer array is instead used, it may be coupled to a beamformer which controls reception of signals by the transducer elements. This can then enable beam steering.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the description and in the claims.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

**Claims**

1. An apparatus for determining a physiological parameter relating to a vessel, the apparatus comprising a processor (36) configured to:

   receive blood flow velocity measurement along a length of the vessel from an ultrasound transducer (16) located at a first location in the vessel;
   receive a pressure measurement from a pressure sensor (14) located at a second location in the vessel;
   receive information on a distance between the first location and a second location along a longitudinal direction of the vessel, said sampling distance;
   determine, based on the sampling distance and the blood flow velocity measurement along the vessel, the blood flow velocity at the second location of the vessel associated to a predetermined time at which the pressure measurement is received;
   derive a physiological parameter characterizing the vessel, based on the pressure measurement and the blood flow velocity at the second location and associated to the predetermined time.

2. The apparatus according to claim 1, wherein the physiological parameter is pulse wave velocity, impedance or resistance.

3. The apparatus according to any preceding claim, wherein the predetermined time is an interval or a discrete time point comprised in a phase of a cardiac cycle, in particular systole.

4. The apparatus according to any preceding claim, comprising:
   at least one intravascular device (20,12) comprising the pressure sensor and the ultrasound transducer.

5. The apparatus according to claim 4, wherein the at least one intravascular device comprises two intravascular devices, in particular a catheter (20) and a guidewire (12) combination, wherein the ultrasound transducer (16) is mounted at a distal portion of the first intravascular device (20) and the pressure sensor (14) is mounted at a distal portion of the second intravascular device (12), which projects beyond the distal end of the first intravascular device.

6. The apparatus according to claim 5, wherein the processor (36) is adapted to set automatically the sampling distance for determining the blood flow velocity at the second location.

7. The apparatus according to claim 5 or 6, wherein each of the intravascular devices comprises at least one marker (22,24), in particular radiopaque or echogenic marker, for alignment of the two intravascular devices along the longitudinal direction.

8. The apparatus according to claim 7, further comprising an extracorporeal imaging device configured to visualize the markers and configured to transmit information to the processor on the locations of the markers with respect to each other.

9. The apparatus according to claim 6, wherein the first intravascular device comprises a sensor configured to detect the sampling distance based on relative motion between the first intravascular device and the second intravascular device.

10. The apparatus according to claim 9, wherein the sensor is the ultrasound transducer and wherein the pressure sensor or its vicinity comprises an echogenic marker.

11. The apparatus according to any of the claims 5 to 10, wherein at least one of the first and second intravascular devices comprises a locking arrangement (28) for constraining relative motion between the first and second intravascular devices.

12. The apparatus according to any of the claims 4 to 11, wherein the ultrasound transducer comprises an annular arrangement of ultrasound transducer elements with active surfaces of the ultrasound transducer elements configured to transmit and receive ultrasound waves to/from direction distal to the intravascular device on which they are mounted.

13. The apparatus according to any of the preceding claims, wherein the physiological parameter comprises pulse wave velocity and wherein the processor is configured to stratify eligibility of a subject to treatment based on a comparison of the pulse wave velocity with a predetermined threshold value.

14. The apparatus according to any of the preceding claims, further comprising a display device (38), wherein the processor is adapted to receive information on the vessel from an imaging device for generating an image of the vessel, and wherein the processor is further adapted to combine the image of the vessel with the physiological parameter representation for display by the display device.

15. A method of determining a physiological parameter relating to a vessel, comprising:

receiving blood flow velocity measurement along a length of the vessel from an ultrasound transducer (16) located at a first location in the vessel;
receiving a pressure measurement from a pressure sensor (14) located at a second location in the vessel;
receiving information on a distance between the first location and a second location along a longitudinal direction of the vessel, said sampling distance;
determining, based on the sampling distance and the blood flow velocity measurement along the vessel, the blood flow velocity at the second location of the vessel associated to a predetermined time at which the pressure measurement is received;
deriving a physiological parameter characterizing the vessel, based on the pressure measurement and the blood flow velocity at the second location and associated to the predetermined time.

FIG. 1

FIG. 2

FIG. 3

FIG. 4

FIG. 5

FIG. 6

FIG. 7

FIG. 8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | J. E. DAVIES: "Use of simultaneous pressure and velocity measurements to estimate arterial wave speed at a single site in humans", AJP: HEART AND CIRCULATORY PHYSIOLOGY, vol. 290, no. 2, 1 January 2005 (2005-01-01), pages H878-H885, XP055006652, ISSN: 0363-6135, DOI: 10.1152/ajpheart.00751.2005 * the whole document * | 1-15 | INV. A61B8/12 A61B8/08 A61B8/06 A61B5/02 A61B5/021 A61B5/0215 A61B8/00 |
| A | WO 2011/110817 A2 (IMP INNOVATIONS LTD [GB]; DAVIES JUSTIN [GB]; MAYET JAMIL [GB]) 15 September 2011 (2011-09-15) * page 7, line 6 - page 11, line 4 * * figures 2,3 * | 1-15 | |
| A | WO 2017/198800 A1 (KONINKLIJKE PHILIPS NV [NL]) 23 November 2017 (2017-11-23) * figures 1a,1b * | 1-15 | |

TECHNICAL FIELDS SEARCHED (IPC)

| T | NABEEL P M ET AL: "Local Pulse Wave Velocity: Theory, Methods, Advancements, and Clinical Applications", IEEE REVIEWS IN BIOMEDICAL ENGINEERING, IEEE, USA, vol. 13, 26 July 2019 (2019-07-26), pages 74-112, XP011766934, ISSN: 1937-3333, DOI: 10.1109/RBME.2019.2931587 [retrieved on 2020-01-17] | | A61B |

The present search report has been drawn up for all claims

| Place of search | Date of completion of the search | Examiner |
|---|---|---|
| Munich | 7 May 2020 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 2768

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2020

| Patent document cited in search report | | Publication date | Patent family member(s) | | Publication date |
|---|---|---|---|---|---|
| WO 2011110817 | A2 | 15-09-2011 | BR | 112012022685 A2 | 22-05-2018 |
| | | | CN | 102905614 A | 30-01-2013 |
| | | | EP | 2544585 A2 | 16-01-2013 |
| | | | ES | 2457268 T3 | 25-04-2014 |
| | | | GB | 2479340 A | 12-10-2011 |
| | | | JP | 5663041 B2 | 04-02-2015 |
| | | | JP | 2013521092 A | 10-06-2013 |
| | | | RU | 2012143201 A | 20-04-2014 |
| | | | WO | 2011110817 A2 | 15-09-2011 |
| WO 2017198800 | A1 | 23-11-2017 | CN | 109152538 A | 04-01-2019 |
| | | | EP | 3457924 A1 | 27-03-2019 |
| | | | JP | 2019516477 A | 20-06-2019 |
| | | | US | 2019090856 A1 | 28-03-2019 |
| | | | WO | 2017198800 A1 | 23-11-2017 |

EPO FORM P0459

For more details about this annex : see Official Journal of the European Patent Office, No. 12/82

# EP 3 831 307 A1

## REFERENCES CITED IN THE DESCRIPTION

**Non-patent literature cited in the description**

- **DAVIES et al.** Use of simultaneous pressure and velocity measurements to estimate arterial wave speed at a single site in humans. *Am J Physiol Heart Circ Physiol,* 2006, vol. 290 (2), H878-85